# EUROPEAN PATENT SPECIFICATION

(11) **EP 2 519 231 B1**
(45) Date of publication and mention of the grant of the patent: **15.03.2017**
(21) Application number: 10807320.6
(22) Date of filing: 01.10.2010
(51) Int. Cl.: A61K 31/69, A61K 45/06, A61P 35/00

(54) **PROTEASOME INHIBITORS FOR TREATING CANCER**
BEHANDLUNG VON ERKRANKUNGEN MIT PROTEASOMHEMMERN
TRAITEMENT DU CANCER PAR DES INHIBITEURS DU PROTÉASOME

(30) Priority: 01.10.2009 US 247714 P
(43) Date of publication of application: 07.11.2012
(73) Proprietor: Janssen Pharmaceutica NV, 2340 Beerse (BE)
(72) Inventor: DE COSTER, Roland, B-2980 Zoersel (BE); VAN DE VELDE, Helgi, B-2470 Retie (BE); BAYSSAS, Martine, CH-1006 Lausannne (CH)
(74) Representative: Warner, James Alexander
(86) International application number: PCT/EP2010/005994
(87) International publication number: WO 2011/038924

(56) References cited:
- WO-A1-2008/086005
- WO-A2-2006/113470
- WO-A2-2009/027644
- ORLOWSKI ROBERT Z ET AL: "Safety and antitumor efficacy of the proteasome inhibitor carfilzomib (PR-171) dosed for five consecutive days in hematologic malignancies: Phase I results", BLOOD, AMERICAN SOCIETY OF HEMATOLOGY, US, vol. 110, no. 11 Part 1, 16 November 2007 (2007-11-16), page 127A, XP008134100, ISSN: 0006-4971
- MUCHAMUEL TONY ET AL: "Preclinical Pharmacology and in Vitro Characterization of PR-047, An Oral Inhibitor of the 20S Proteasome", BLOOD, AMERICAN SOCIETY OF HEMATOLOGY, US, vol. 112, no. 11, 16 November 2008 (2008-11-16), page 1257, XP008134101, ISSN: 0006-4971
- BROSS PETER F ET AL: "Approval summary for bortezomib for injection in the treatment of multiple myeloma", CLINICAL CANCER RESEARCH, THE AMERICAN ASSOCIATION FOR CANCER RESEARCH, US, vol. 10, no. 12 Pt 1, 15 June 2004 (2004-06-15), pages 3954-3964, XP002554400, ISSN: 1078-0432
- CRESTA S ET AL: "Phase I study of bortezomib with weekly paclitaxel in patients with advanced solid tumours", EUROPEAN JOURNAL OF CANCER, PERGAMON PRESS, OXFORD, GB, vol. 44, no. 13, 1 September 2008 (2008-09-01), pages 1829-1834, XP023979218, ISSN: 0959-8049, DOI: DOI:10.1016/J.EJCA.2008.05.022 [retrieved on 2008-07-17]

## Description

### Field of the Invention

The proteasome inhibitor bortezomib is provided for treating cancer in patients in need thereof.

### Description of the Related Art

In eukaryotic cells, the ubiquitin-proteasome pathway is the central pathway for protein degradation of intracellular proteins. Proteins are initially targeted for proteolysis by the attachment of a polyubiquitin chain, then rapidly degraded to small peptides by the proteasome and the ubiquitin is subsequently released and recycled. This coordinated proteolytic pathway is dependent upon the synergistic activity of the ubiquitin- conjugating system and the 26S proteasome. The 26S proteasome is a large, approximately 1500 to 2000 kDa, multi-subunit complex present in the nucleus and cytoplasm of eukaryotes. The catalytic core of this complex, referred to as the 20S proteasome, is a cylindrical structure consisting of four heptameric rings containing α- and β- subunits. The proteasome is a threonine protease, the N-terminal threonine of the β-subunit providing the nucleophile that attacks the carbonyl group of the peptide bond in target proteins. At least three distinct proteolytic activities are associated with the proteasome: chymotryptic, tryptic and peptidylglutamyl. The ability to recognize and bind polyubiquinated substrates is conferred by 19S (PA700) subunits, which bind to each end of the 20S proteasome. These accessory subunits unfold substrates and feed them into the 20S catalytic complex, whilst removing the attached ubiquitin molecules. Both the assembly of the 26S proteasome and the degradation of protein substrates are ATP-dependent (Almond, Leukemia, 16: 433, 2002).

Exposure of a variety of tumor-derived cell lines to proteasome inhibitors triggers apoptosis, likely resulting from effects on several pathways, including cell cycle regulatory proteins, p53, and nuclear factor kappa B (NF-κB). Many of the initial studies documenting proteasome inhibitor-mediated apoptosis used cells of hematopoietic origin, including monoblasts, T-cell and lymphocytic leukemia cells, lymphoma cells, and promyelocyte leukemia cells. The first demonstration of *in vivo* antitumor activity of a proteasome inhibitor used a human lymphoma xenograft model. Furthermore, proteasome inhibitors were reported to induce preferential apoptosis of patient-derived lymphoma and leukemia cells and to preferentially inhibit proliferation of multiple myeloma cells with relative sparing of control, non-transformed cells. Thus, proteasome inhibitors are particularly useful as therapeutic agents in patients with refractory hematologic malignancies.

One such proteasome inhibitor is bortezomib, also known as PS-341 (Velcade® developed by Millennium Pharmaceuticals, Inc, Cambridge, MA). Bortezomib is a dipeptide boronic acid derivative and a highly selective, potent, reversible proteasome inhibitor with a Kᵢ of about 0.6 nmol/L (Adams et al., Semin Oncol 28:613-619, 2001). Bortezomib has been studied extensively for its effect on various cellular functions requiring the ubiquitin-proteasome pathway in both *in vitro* and *in vivo* systems. WO2006/113470 discloses the administration of telomerase inhibitor GRN163L in combination with bortezomib but does not address the administration of bortezomib alone.

Bortezomib is currently approved in several parts of the world for the treatment of hematologic cancers. For example, bortezomib is approved in the U.S. for the treatment of patients with multiple myeloma as a first line treatment or with mantle cell lymphoma who have received at least one prior therapy. The present regimen for treating patients is dosing bortezomib intravenously bi-weekly or weekly. Under the currently approved dosing, at least 72 hours must elapse between bortezomib administrations. Recently, preclinical and clinical studies have indicated that subcutaneous bortezomib dosing is a valid alternative for intravenous administration.

Biological studies have shown that key aberrant signaling pathways impacted by proteasome inhibition in hematological cancers are also present in solid tumors, suggesting that proteasome inhibition may also be useful for treatment of solid tumors. However, one of the major toxicities associated with bortezomib therapy is peripheral neuropathy. Reduction in dosage or frequency of bortezomib administration is indicated in cases where peripheral neuropathy symptoms are present. This side effect is a major obstacle in administering more frequent, higher or alternate dosing of bortezomib, which may be required to achieve therapeutic benefit for other types of cancers, including solid tumors.

Therefore, it is an objective of the present application to provide alternative dosage regimens for proteasome inhibitors, including bortezomib, which result in improved therapy for patients with cancer.

### Summary of the Invention

The present invention provides the proteasome inhibitor bortezomib for use in a method for treating cancer in a patient, comprising administering to said patient multiple doses of said bortezomib, wherein said multiple doses are administered at least four days a week for at least two weeks. Also disclosed herein is a use in a method for treating a proteasome mediated or related disorder in a patient, comprising administering to the patient a proteasome inhibitor more frequently than every 72 hours, or at least three days a week, at least four days a week, at least five days a week, at least six days a week or seven days a week. Also disclosed herein is a use of a proteasome inhibitor for treating a proteasome mediated or related disorder in a patient wherein the proteasome inhibitor is administered to the patient more frequently than every 72 hours, or at least three days a week, at least four days a week, at least five days a week, at least six days a week or seven days a week. The proteasome inhibitor may be administered for any length of time, including three days, one week, two weeks, three weeks, four weeks, five weeks, six weeks, seven weeks, eight weeks, up to several months and years. The proteasome inhibitor also may be administered in treatment cycles comprising a treatment period during which the proteasome inhibitor is administered and a rest period during which no proteasome inhibitor is administered.

The proteasome mediated or related disorder may be cancer. The proteasome inhibitor may be bortezomib.

The use may further comprise an additional treatment. The additional treatment may be an additional anti-cancer treatment.

Other objects and features of the present invention will become apparent from the following detailed description considered in conjunction with the accompanying drawings. It is to be understood, however, that the drawings are designed solely for purposes of illustration and not as a definition of the limits of the invention, for which reference should be made to the appended claims. It should be further understood that the drawings are not necessarily drawn to scale and that, unless otherwise indicated, they are merely intended to conceptually illustrate the structures and procedures described herein.

### Brief Description of the Drawings

In the drawings:

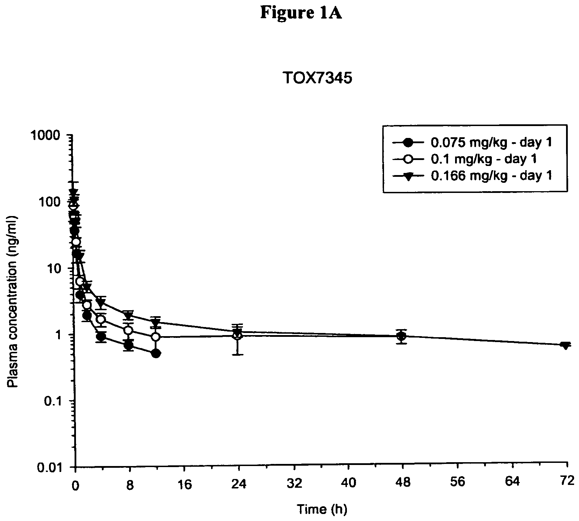

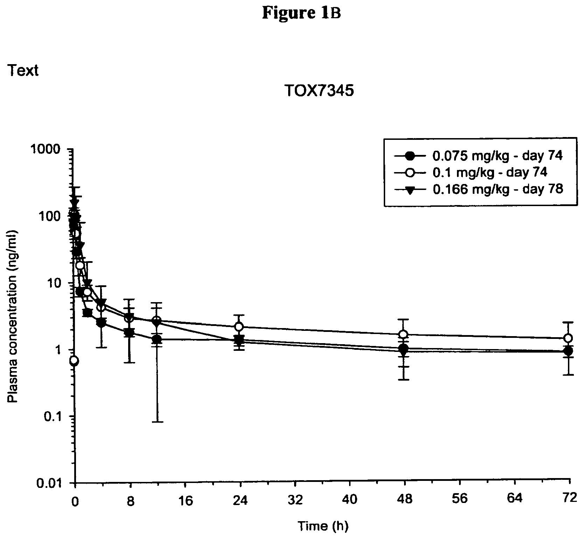

Fig 1A and 1B: Plasma concentrations of bortezomib in Cynomolgus monkeys after single (day 1) and repeated (day 74, 0.075 and 0.1 mg/kg or day 78, 0.166 mg/kg) subcutaneous administration of bortezomib at about 0.075, 0.1 and 0.166 mg/kg in a bi-weekly (0.075 and 0.1 mg/kg) or weekly (0.166 mg/kg) dosing regimen (TOX7345).

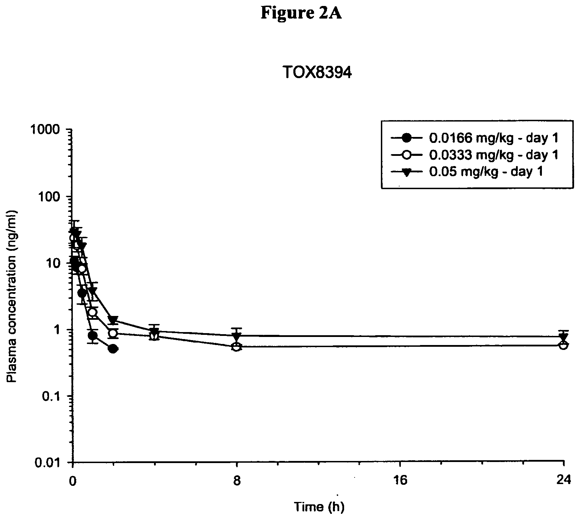

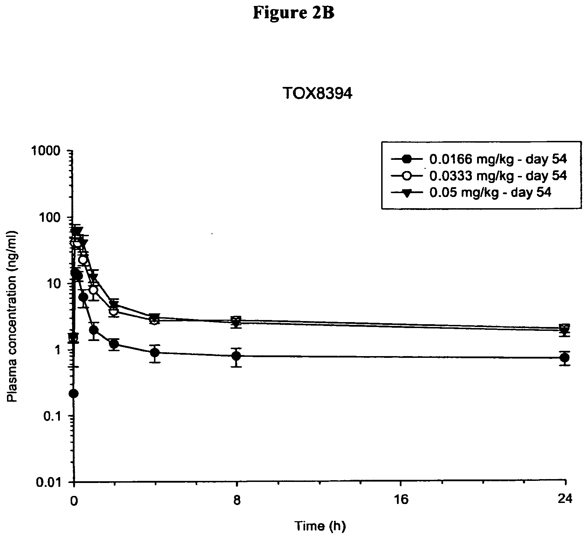

Fig 2A and 2B: Plasma concentrations of bortezomib after single (day 1) and repeated (day 54) subcutaneous administration of bortezomib at about 0.0166, 0.0333 and 0.05 mg/kg in a daily (5/7days) dosing regimen for 8 consecutive weeks in Cynomolgus monkeys (TOX8394).

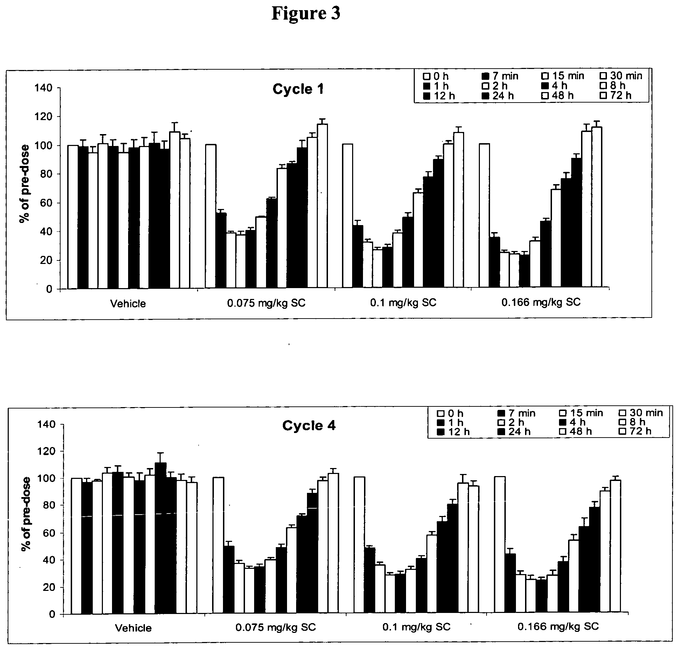

Figure 3: Proteasome inhibition by bortezomib after single (day 1, cycle 1) and repeated (day 74, about 0.075 and 0.1 mg/kg, (cycle 4) or day 78, about 0.166 mg/kg) subcutaneous administration of bortezomib at about 0.075, 0.1 and 0.166 mg/kg in a bi-weekly (about 0.075 and 0.1 mg/kg) or weekly (about 0.166 mg/kg) dosing regimen in Cynomolgus monkeys (TOX7345).

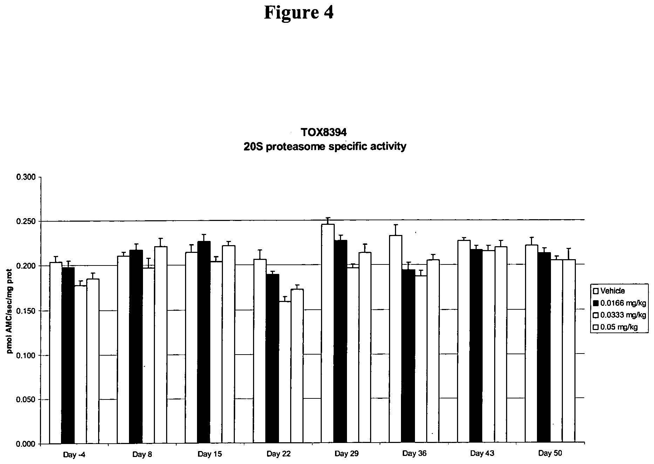

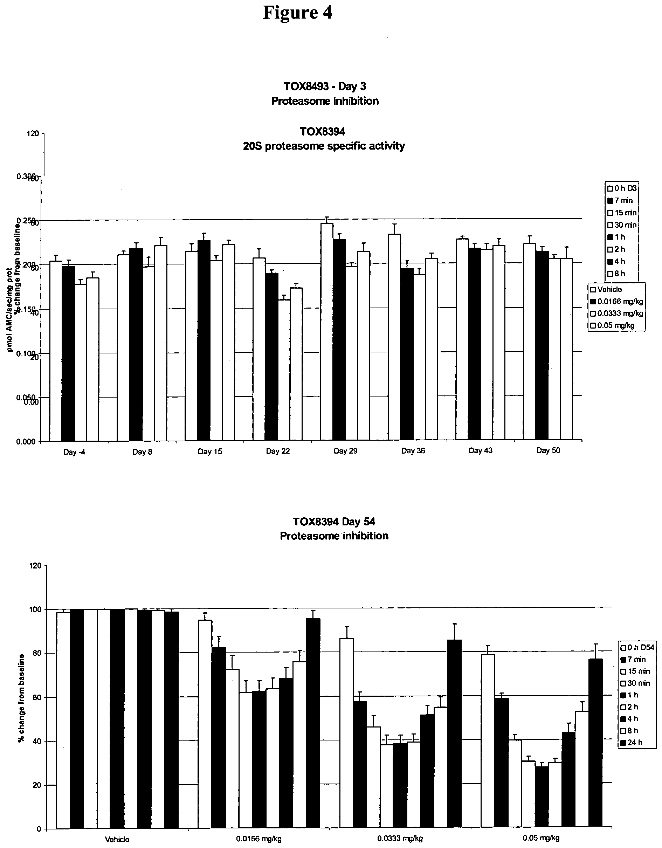

Figure 4: Proteasome inhibition in whole blood by bortezomib on day 3 and 54 of subcutaneous administration of bortezomib at about 0.0166, 0.0333 and 0.05 mg/kg in a daily (5/7days) dosing regimen for 8 consecutive weeks in Cynomolgus monkeys. The first graph shows the Specific Activity before dosing each week, i.e., after a 2 day recovery period (TOX8394).

### Detailed Description of the Presently Preferred Embodiments

Disclosed herein is a use in a method for treating a proteasome mediated or related disorder in a patient in need thereof comprising administering to the patient multiple doses of an effective amount of a proteasome inhibitor, wherein said multiple doses are administered more frequently than every seventy-two hours or at least three days a week.

It is found herein that administering bortezomib at least three days per week maintains similar pharmacodynamic effectiveness and safety profiling as administering bi-weekly. Even when the dose administered at least three days per week results in a higher cumulative weekly dose and higher cumulative proteasome inhibition than a dose administered bi-weekly, the safety profile including peripheral neuropathy and local tolerance is comparable between the two regimens. This result is surprising and unexpected because it was previously believed that less frequent dosing was necessary to achieve an acceptable safety profile. The present disclosure expands the use of proteasome inhibitors for therapy.

In certain embodiments, the proteasome mediated or related disorder is graft versus host disease (GVHD). In other embodiments, the proteasome mediated or related disorder is a condition related to aberrant cell growth. Conditions related to aberrant cell growth include metaplasias, dysplasias, hyperplasias and neoplasias. Neoplasias include cancer.

Examples of cancer which may be treated according to the present invention include both solid tumors, such as carcinomas, adenocarcinomas, sarcomas; and non-solid tumors, including cancers of hematological origin. Cancers of hematological origin include leukemias, such as acute myelogenous leukemia, chronic myelognous leukemia, lymphocytic leukemia, and myeloid leukemia; lymphomas such as mantle cell lymphoma, cutaneous T-cell lymphoma, Hodgkin's lymphoma and non-Hodgkin's lymphoma; and myelomas, such as multiple myeloma. Solid tumors include tumors of the skin (such as melanoma), lung, bronchus, prostate, breast, pancreas, adrenal gland, small intestine, large intestine, colon, rectum, thyroid, stomach, liver, bile duct, kidney, renal pelvis, urinary bladder, uterus, cervix, ovary, esophagus, brain, head and neck, oral cavity, pharynx, and larynx. Specific cancers include myxoid and round cell carcinoma, locally advanced tumors, human soft tissue carcinoma, cancer metastases, squamous cell carcinoma, esophageal squamous cell carcinoma, cancer of the adrenal cortex, ACTH-producing tumors, nonsmall cell cancers, breast cancer, gastrointestinal cancers, urological cancers, malignancies of the female genital tract, malignancies of the male genital tract, kidney cancer, brain cancer, bone cancers, skin cancers, thyroid cancer, retinoblastoma, neuroblastoma, peritoneal effusion, malignant pleural effusion, mesothelioma, Wilms's tumors, gall bladder cancer, trophoblastic neoplasms, hemangiopericytoma, and Kaposi's sarcoma.

The treatment may be first-line, second-line, third-line, fourth-line or further lines. The cancer may be recurrent, or refractory to one or more anti-cancer treatments. Thus, the patient may be treatment-naive, or may have received one or more previous anti-cancer therapies which did not completely cure the disease.

The term "patient" means an animal, who is or has been the object of treatment, observation or experiment and is at risk of (or susceptible to) developing a disease or disorder or having a disease or disorder related to unregulated proteasome activity. Animals include mammals, such as humans, apes, monkeys, cows, horses, rabbits, rats, or mice. In certain embodiments, the patient is a human patient.

By "treating" a disorder or disease, it is meant that the administration will result in prevention, inhibition in the rate of advancement, amelioration, or complete cure of one or more symptoms of the disease or disorder. The term also indicates the amount that could effectively enhance physiological function. For cancer treatment, treatment includes reduction in the number of cancer cells; reduction in tumor size; reduction in tumor number; inhibition of (i.e., slow to some extent and preferably stop) cancer cell infiltration into peripheral organs; inhibition of (i.e., slow to some extent and preferably stop) tumor metastasis; inhibition of tumor growth; and/or reduction in one or more of the symptoms associated with the cancer. To the extent the active ingredient or agent may prevent growth and/or kill existing cancer cells, it may be cytostatic and/or cytotoxic. For cancer therapy, efficacy can, for example, be measured by assessing the time to disease progression (TTP) and/or determining the response rate (RR).

The term "administering," with respect to the methods of the present invention, refers to a means for treating a disease as described herein with a compound specifically disclosed or a compound or prodrug thereof, which would be included within the scope of the invention albeit not specifically disclosed for certain of the instant compounds.

Proteasome inhibitors useful in the disclosure include peptide aldehydes and peptide vinyl sulfones, which bind to and directly inhibit active sites within the 20S core of the proteasome. Peptide aldehydes and peptide vinyl sulfones bind to the 20S core particle in an irreversible manner, hence the proteolytic activity cannot be restored upon their removal.

Peptide boronates or peptide boronic acid compounds are also proteasome inhibitors, but function differently from the peptide aldehyde analogs. They confer stable inhibition of the proteasome and dissociate slowly from the proteasome. Further, the peptide boronic acid compounds are more potent than their peptide aldehyde analogs, and act more specifically in the weak interaction between boron and sulfur. Therefore, peptide boronates do not inhibit thiol proteases (Richardson et al., Cancer Control 10: 361, 2003).

Peptide boronic acid compounds are peptides containing an α-aminoboronic acid at the acidic end or C-terminal end of the sequence (Zembower et al., lnt. J. Pept. Protein Res. 47: 405-413, 1996). Due to the ability to form a stable tetrahedral borate complex between the boronic acid group and the active site serine or histidine moiety, peptide boronic acids are powerful serine-protease inhibitors. This activity is often enhanced and made highly specific towards a particular protease by varying the sequence of the peptide boronic acids and introducing unnatural amino acid residues and other substituents. Peptide boronic acid compounds and their synthesis have been described previously, including U.S. Patent Numbers 4,499,082; 4,537,773; 5,780,454; 6,083,903; 6,297,217; 6,617,317; 6,713,446; and 6,958,319.

Peptide boronic acid compounds generally have the form below: 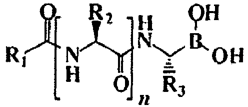 wherein R¹, R², and R³ are independently selected moieties that can be the same or different from each other, and n is from 1-8, preferably 1-4.

Preferably, R¹, R², and R³ are independently selected from hydrogen, alkyl, alkoxy, aryl, aryloxy, aralkyl, aralkoxy, cycloalkyl, or heterocycle, or any of R¹, R², and R³ may form a heterocyclic ring with an adjacent nitrogen atom in the peptide backbone. Alkyl, including the alkyl component of alkoxy, aralkyl and aralkoxy, is preferably 1 to 10 carbon atoms, more preferably 1 to 6 carbon atoms, and may be linear or branched. Aryl, including the aryl component of aryloxy, aralkyl, and aralkoxy, is preferably mononuclear or binuclear (i.e. two fused rings), more preferably mononuclear, such as benzyl, benzyloxy, or phenyl. Aryl also includes heteroaryl, i.e. an aromatic ring having one or more nitrogen, oxygen, or sulfur atoms in the ring, such as furyl, pyrrole, pyridine, pyrazine, or indole. Cycloalkyl is preferably 3 to 6 carbon atoms. Heterocycle refers to a non-aromatic ring having one or more nitrogen, oxygen, or sulfur atoms in the ring, preferably a 5- to 7-membered ring having included 3 to 6 carbon atoms. Such heterocycles include, for example, pyrrolidine, piperidine, piperazine, and morpholine. Either of cycloalkyl or heterocycle may be combined with alkyl, e.g. cyclohexylmethyl.

Any of the above groups (excluding hydrogen) may be substituted with one or more substituents selected from halogen, preferably fluoro or chloro, hydroxy, lower alkyl, lower alkoxy, such as methoxy or ethoxy, keto, aldehyde, carboxylic acid, ester, amide, carbonate, or carbamate, sulfonic acid or ester, cyano, primary, secondary, or tertiary amino, nitro, amidino, and thio or alkylthio. Preferably, the group includes at most two such substituents. Specific examples of R¹, R², and R³ include n-butyl, isobutyl, and neopentyl (alkyl), phenyl or pyrazyl (aryl), 4-((t-butoxycarbonyl) amino)butyl,3-(nitroamidino)propyl, and (1-cyclopentyl-9-cyano)nonyl (substituted alkyl), naphthylmethyl and benzyl (aralkyl), benzyloxy (aralkoxy), and pyrrolidine (R² forms a heterocyclic ring with an adjacent nitrogen atom). For R1, preferred groups are substituted and unsubstituted aryl and heteroaryl. For R2 and R3, preferred groups are natural amino acid side chains (e.g., alkyl, hydroxyalkyl, aralkyl, heteroaralkyl, aminoalkyl, carboxyalkyl, guanidinoalkyl) or variants thereof.

In general, the peptide boronic acid compound can be a mono-peptide, di- peptide, tripeptide, or a higher order peptide compound. Other peptide boronic acid compounds are described in U S Patent Numbers 6,083,903; 6,297,217; and 6,617,317. Compounds having an aspartic acid or glutamic acid residue with a boronic acid as a side chain are also included.

In the invention, the proteasome inhibitor is bortezomib. Bortezomib, also called [(1R)-3-methyl-1-[[(2S)-1-oxo-3-phenyl-2-[pyrazinylcarbonyl)amino] propyl]amino] butyl] boronic acid or N-pyrazine carbonyl-L-phenylalanine-L-leucine boronic acid, is a modified dipeptidyl boronic acid derived from leucine and phenylalanine.

Other proteasome inhibitors useful in the present disclosure include NPI-0052, carfilzomib, ritonavir, disulfiram, Salinosporamide A, epigallocatechin-3-gallate, genistein and curcumin.

The proteasome inhibitor is administered more frequently than every 72 hours, or at least three times a week. The proteasome inhibitor may be administered every 6-60 hours, including every 48 hours, every 24 hours, or every 12 hours. The proteasome inhibitor may be administered three, four, five, six or seven days a week, and may be administered once daily or multiple times a day. The administration may be at regular intervals, or the intervals between administration may vary. The administration may be on consecutive days, or non-consecutive days. There may be periods of rest with no proteasome inhibitor administration
For example, for administration three times a week, administration may be on day 1, day 2, day 3 with rest on days 4-7; or on day 1, day 3, day 4 with rest on days 5-7; or on day 1, day 3, and day 5, with rest on days 6-7 etc. In certain embodiments, the proteasome inhibitor is administered on days 1-5, with no proteasome inhibitor administered on days 6-7.

Treatment may continue for as long as necessary, and may be as short as one week. In certain embodiments, treatment continues for at least two weeks, at least three weeks, at least four weeks, at least five weeks, at least six weeks, at least seven weeks, at least eight weeks or more. The administration regime and dosage may be the same week to week or may vary week to week.

The proteasome inhibitor can be administered in treatment cycles, with a rest between cycles. For example, the proteasome inhibitor can be administered five days per week on days 1-5 and 8-12, with a rest of one week or two weeks before repeating the cycle.

The proteasome inhibitor is typically at a dose of from about 0.001 mg to about 300 mg/kg of body weight per day; from about 0.03 to about 100 mg/kg of body weight per day; or from about 0.05 to about 15 mg/kg of body weight per day. Optimal dosages will vary depending on factors associated with the particular patient being treated (e.g., age, weight, diet and time of administration), the severity of the condition being treated, the compound being employed, the mode of administration and the strength of the preparation. Each dose may be the same, or the dose may vary from administration to administration, day to day, or week to week.

In certain embodiments, the proteasome inhibitor is administered at doses of about 0.1, 0.2, 0.4, 0.5, 0.6, 0.7, 0.8, 0.9, 1.0, or 1.1 mg/m². By way of example, a treatment comprises administering bortezomib once daily at a dose of about 0.0166, 0.0333 or 0.05 mg per kg of body weight (e.g. about 0.2, 0.4 or 0.6 mg/m²). The once daily dosing regimen generates similar pharmacodynamic effect without increasing toxicological effects as compared to a biweekly dosing regimen of administering bortezomib at about 0.075 or 0.1 mg per kg body weight (e.g. about 0.9 or about 1.2 mg/m²) or a weekly dosing regimen of administering bortezomib at about 0.166 mg/kg (e.g. about 2.0 mg/m²).

In certain embodiments, the use includes administering one or more additional treatments. The additional treatment may be administered to alleviate a side effect of the proteasome inhibitor, to alleviate another symptom or aspect of the proteasome mediated or related disease, to augment the effectiveness of the proteasome inhibitor, , or to treat an unrelated disease or disorder.

Additional treatments useful in the present invention include anti-cancer treatments, immunosuppressants, anti-viral agents, anti-inflammatory agents, anti-fungal agents, antibiotics, anti-vascular hyperproliferation agents, anti-depressive agents, hypolipidemic agents or lipid-lowering agents or lipid modulating agents, antidiabetic agents, anti-obesity agents, antihypertensive agents, platelet aggregation inhibitors, and/or anti-osteoporosis agents.

Additional anti-cancer treatments useful in the present invention include surgery, chemotherapy, radiation therapy, hormonal therapy, gene therapy, antibody therapy, and immunotherapy. Such combinational treatment may be achieved by simultaneous, sequential or separate administration of the divided or combined treatments. By way of example, the combination therapy may comprise co-administration of a peptide boronic acid compound or composition thereof and a chemotherapeutic agent, sequential administration of a peptide boronic acid compound or composition thereof and a chemotherapeutic agent, administration of a composition containing a peptide boronic acid compound and a chemotherapeutic agent, or simultaneous administration of a separate composition containing a peptide boronic acid compound and a separate composition containing a chemotherapeutic agent.

Chemotherapeutic agents useful in the present invention include anti-angiogenic agents, anti-tumor agents, cytotoxic agents, inhibitors of cell proliferation, and the like or mixtures thereof. Such chemotherapy may cover three main categories of therapeutic agent:
1. Cell-cycle specific chemotherapeutic agents including, but not limited to, epipodophyllotoxins (e.g. etoposide and teniposide); diterpenoids (e.g. paclitaxel and docetaxel); vinca alkaloids (e.g. vincristine, vindesine, vinblastine, and vinorelbine); antimetabolites (e.g. cladrabine, cytarabine, allopurinol, fludurabine, methotrexate, mercaptopurine, thioguanine, 5-fluorouracin and fluorodeoxyuridine) and camptothecins (e.g. 9-amino camptothecin, topotecan and irinotecan).
2. Cytotoxic chemotherapeutic agents including, but not limited to, alkylating agents (e.g. hexamethylmelamine, busulfan, melphalan, chlorambucil, cyclophosphamide, mechlorethamine, carmustine, lomustine, dacarbazine, carboplatin, displatin and oxaliplatin); antitumor antibiotics (e.g. bleomycin, idarubicin, mitomycin-c, doxorubicin, daunomycin, epirubicin, dactinomycin and mithramycin);
3. Other anticancer agents including, but not limited to, testosterone 5α-dihydroreductase inhibitors (e.g. finasteride); anti-estrogens (e.g. tamoxifen, toremifent, raloxifene, droloxifene and iodoxyfene); progestrogens (e.g. megestrol acetate); aromatase inhibitors(e.g. anastrozole, letrazole, vorazole, and exemestane; antiandrogens (e.g. flutamide, nilutamide, bicalutamide, and cytorterone acetate); LHRH agonists and antagonists (e.g. goserelin acetate and luprolide); metalloproteinase inhibitors (e.g. marimastat); urokinase plasminogen activator receptor function inhibitors; cyclooxygenase type 2 inhibitors (e.g. celecoxib); angiogenesis inhibiting agents such as VEGFR and TIE-2 inhibitors; growth factor function inhibitors such as inhibitors of hepatocyte growth factor; erb-B2, erb-B4, epidermal growth factor receptor (e.g. Iressa and Tarceva), platelet derived growth factor receptor, vascular endothelial growth factor receptor and TIE-2; and other tyrosine kinase inhibitors other than those described in the present invention.

Most commonly used chemotherapeutic agents include taxanes (including paclitaxel and docetaxel), camptothecin, doxorubicin, bohemine, methotrexate, platinum-based therapies including cisplatin, oxaliplatin, carboplatin, topoisomerase inhibitors (including etoposide and topotecan) and podophyllotoxin.

In certain embodiments, the one or more additional anticancer therapies are melphalan and/or prednisone; dexamethasone, cyclophosphamide, and/or lenalidomide; romidepsin; gemcitabine; irinotecan; docetaxel; or doxorubicin.

The proteasome inhibitor(s) or additional treatments may be administered by any suitable administration route. Suitable administration routes include oral and parenteral, including intravenous, subcutaneous, intramuscular, intradermal, and transdermal. The compounds may be administered via continuous infusion or as a single bolus. In certain embodiments, the proteasome inhibitor is administered subcutaneously.

The proteasome inhibitor(s) or additional treatments may be administered as part of a pharmaceutical formulation. Pharmaceutical formulations may be prepared by any of the conventional and unconventional pharmaceutical methods well known in the pharmacy art and may be adapted for administration by any appropriate route.

Pharmaceutical formulations adapted for subcutaneous administration may include aqueous and non-aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents. Formulations for transdermal administration may be presented as discrete patches intended to remain in intimate contact with the epidermis of the recipient for a prolonged period of time.

The invention is not limited by the embodiments described above which are presented as examples only but can be modified in various ways within the scope of protection defined by the appended patent claims.

### EXAMPLE 1. PRECLINICAL STUDIES METHODS AND MATERIALS

### Study Design

For bi-weekly dosing treatment (TOX 7345), a group of 3 male and 3 female Cynomolgus monkeys were dosed for 4 cycles. Each cycle consisted of dosing with vehicle or with bortezomib at about 0.075 or about 0.1 mg/kg (e.g. about 0.9 or about 1.2 mg/m²) on days 1, 4, 8 and 11 followed by 1 week rest period. A second group of 3 male and 3 female Cynomolgus monkeys were dosed with bortezomib weekly at about 0.166 mg/kg (e.g., about 2.0 mg/m²) for 12 consecutive weeks. For daily dosing treatment (TOX 8394), a group of 3 male and 3 female Cynomolgus monkeys were dosed with vehicle or bortezomib at about 0.0166, 0.0333 or 0.05 mg/kg (e.g. about 0.2, 0.4 or 0.6 mg/m²) for 5 consecutive days followed by 2 days of rest period for 8 consecutive weeks.

The test substance and vehicle were freshly prepared on the day of use. Bortezomib was provided in pre-weighed vials each containing about 3.5 mg of bortezomib and about 35 mg of mannitol. The substance was reconstituted by adding of about 0.9 % w/v Sodium Chloride for Injection USP and by gently mixing to provide a solution of bortezomib of about 3.5 (TOX7345) or 1 (TOX8394) mg/ml. The vehicle for control animals was prepared by dissolving an appropriate quantity of mannitol in an appropriate volume of about 0.9 % w/v Sodium Chloride for Injection USP to provide a dosing solution containing mannitol at a concentration equal to that contained in the 3.5 mg/ml (TOX7345) or 1.0 mg/ml (TOX8394) bortezomib dosing solution.

Each group was evaluated for toxicological profile, pharmacokinetics and pharmacodynamics as described below.

### EXAMPLE 2. PRECLINICAL SCREENING STUDIES

For each study group, the following toxicological screening was performed: mortality, body weight, food consumption, clinical observations, ophthalmology, electrocardiography, hematology, serum biochemistry, urinalysis, injection site evaluations (erythema and edema), neurological examinations, histology, and peripheral neuropathy. The results of the toxicology screening or safety evaluation were summarized in Table 1 below.

**Table 1. Results of Toxicological Screening for the bi-weekly and the once daily dosing regimens.**

| **Parameter** | **TOX 7345 (bi-weekly dosing)** | **TOX 8394 (Once daily dosing)** |
|---|---|---|
| Mortality | 1 F (day 72), 1 M (day 79), at 0.166 mg/kg (weekly) considered to be treatment related | No mortality at any dose level |
| | No mortality at other dose levels | |
| Body weight | Body weight loss only in 2/3 M (0.166 mg/kg) | Body weight loss at 0.05 mg/kg (n=2/3 M) |
| | No body weight loss at other dose levels | No body weight loss at any other dose level |
| Clinical observations | Abnormal faeces (soft, liquid, and/or mucoid) and emesis (0.166 (weekly) and 0.1 mg/kg) Reduced appetite and decreased activity (0.166 (weekly) and 0.1 mg/kg) | Abnormal faeces (soft, liquid, 0.0333 or 0.05 mg/kg) |
| Haemathology | ↑ WBC (0.166 mg/kg) in decedent female monkey | Platelet numbers decreased and mean platelet volume increased (across all bortezomib treated groups in a dose-dependent manner) |
| | No hematological changes related to bortezomib at other dose levels | |
| Serum biochemistry | ↓ Total protein and globulin (0.166 (weekly) and 0.1 and 0.075 mg/kg) (may be secondary to reduced appetite) | No changes in serum chemistry |
| Histology | BONE MARROW: hypocellularity 0.166 (weekly), 0.1, 0.075 mg/kg (frequency and dose dependent) | BONE MARROW : no findings |
| | | LYMPH NODES: no findings |
| | LYMPH NODES: lymphoid atrophy (0.166 (weekly), 0.1 mg/kg) | SPLEEN: no findings |
| | SPLEEN: pulp, lymphoid atrophy (0.166 (weekly); 0.1 mg/kg) | KIDNEY: tubular degeneration : 0.0333 and 0.05 mg/kg |
| | KIDNEY: renal cortical tubules degeneration/hypertrophy (0.166 (weekly), 0.1, 0.075 mg/kg) | PANCREAS: decreased zymogen, granules 0.0333 and 0.05 mg/kg |
| | | PROSTATE: necrotic debris, 0.0333 and 0.05 mg/kg |
| | PANCREAS: no findings | |
| | PROSTATE: no findings | |
| Injection site | No significant signs of irritation or treatment- related microscopic changes | Dose dependent increase in incidence, frequency and severity of erythema and oedema. Transient in nature |
| | | Dose related increase in number of areas and histological sections affected by mononuclear cell neutrophil infiltration (minimal to mild) |
| Peripheral neuropathy | 0.075 mg/kg no peripheral nerve lesions | 0.0166 mg/kg: no neuronal lesions |
| | 0.1/0.166 (weekly) mg/kg minimal focal axonal neurofiber degeneration | 0.0333- 0.05 mg /kg: |
| | | Spinal cord: dorsal funiculi (cervical & lumbar) minimal degeneration |
| | Spinal cord (dorsal root ganglia) | Peripheral (sciatic, tibial and peroneal nerves): minimal degeneration |
| Electrocardiography | No abnormalities found at any dose level | No abnormalities found at any dose level |
| Ophthalmology | No treatment related changes | No treatment related changes |
| | | |
| Urinalysis | No treatment related findings | No treatment related findings |

As shown in Table 1, repeated, once-daily, five times per week subcutaneous administration of bortezomib was well tolerated by Cynomolgus monkeys for eight weeks. All monkeys survived to scheduled sacrifice. No bortezomib-related changes were found in ECG, ophthalmology, rectal temperature or blood pressure results. Also, no gross abnormalities were observed in bortezomib-treated monkeys at necropsy. No changes in serum chemistry parameters resulted from the treatment.

Draize scores of the injection site showed a dose-dependent increase with the bortezomib treatment in incidence, frequency and severity of erythema and edema that correlated with the histopathological finding of fibroplasia at the injection sites in male and female monkeys administered at about 0.05 mg/kg/dose. There was a dose-related increase in the number of areas and histological sections affected by mononuclear cell/neutrophil infiltration (minimal to mild) at injection sites.

Mild inappetence was observed in one male and one female administered at about 0.0333 mg/kg/dose and one female administered at about 0.05 mg/kg/dose. However, nine male and female monkeys in these groups lost about 3 - 12% of body weight. All but two male monkeys administered at about 0.05 mg/kg/dose regained body weight during the dosing period. Soft stool was also noted on multiple days for one male monkey in each group of administered at about 0.0166 mg/kg/dose and 0.0333 mg/kg/dose, and one female monkey administered at about 0.05 mg/kg/dose.

Platelet numbers decreased, mean platelet volume increased and fibrinogen concentrations increased across all bortezomib-treated groups in a dose-dependent manner. Increases in the urinalysis parameters creatinine, inorganic phosphorus, ketones, protein and sodium correlated with pathology findings. Kidney weights in all monkeys administered at about 0.05 mg/kg/dose were greater than kidney weights of control monkeys. Furthermore, tubular degeneration/regeneration was observed in the kidneys of male monkeys administered at about 0.0333 mg/kg/dose and in male and female monkeys administered at about 0.05 mg/kg/dose.

Other histopathological findings were decreased, zymogen granules in the pancreas in male and female monkeys administered at about 0.0333 or 0.05 mg/kg/dose and necrotic debris in acini of the prostate of male monkeys administered at about 0.0333 and 0.05 mg/kg/dose. The MTD was about 0.1 mg/kg in the bi-weekly dosing regimen (TOX7345) and about 0.05 mg/kg in the daily dosing regimen (TOX8394).

In summary, subcutaneous administration of bortezomib at >= 0.0166 mg/kg/dose resulted in changes in various urinalysis parameters, decreased platelets, and increased mean platelet volume and serum fibrinogen concentration. Subcutaneous administration of bortezomib at >= 0.0333 mg/kg/dose resulted in decreased food consumption, and lesions in the kidney, pancreas and prostate, and initial body weight loss that in most cases was followed by body weight gain during the course of treatment. At the high dose of about 0.05 mg/kg/dose, histopathologic lesions at the injection site and kidney weights were higher than controls. In addition, two males with initial weight loss did not regain weight over the course of treatment. Therefore, under conditions of the study and based on the available data, the 'No Observed Adverse Effect Level' (NOAEL) for repeated, five times weekly subcutaneous administration of bortezomib to Cynomolgus monkeys for 8 weeks cannot be identified.

### EXAMPLE 3. PRECLINICAL TOXICOKINETICS AND PHARMACOKINETICS

For each study, the values of the time to maximum plasma concentration (Tₘₐₓ), the maximum plasma concentration (Cₘₐₓ) were determined by visual inspection of the data. The area-under-the plasma-concentration-time curve (AUC) was estimated by the linear trapezoidal rule. Plasma samples were analyzed using a validated LC/MS/MS method with a lower quantification limit of about 0.5 ng/ml.

The plasma concentrations of bortezomib in the bi-weekly and the once daily dosing regimens were shown in Fig 1A, 1B, 2A, 2B, respectively. There were no differences in exposure between male and female monkeys. Therefore, mean parameters were calculated using results obtained from both male and female animals.

In each experiment, bortezomib was rapidly absorbed after dosing. The value of Tₘₐₓ occurred on average at 0.25 h after dosing at the latest. The values of Cₘₐₓ and AUC increased with increasing dose levels of bortezomib either proportionally or slightly more than dose proportionally for the bi-weekly as well as for the daily dosing regimens.

Further, in each study, the exposure (AUC) increased upon repeated administration. The increase was more significant from the first to the early repeated dose administrations than between the later dose administrations. The data indicates that, from the second cycle in the bi-weekly treatment regimen (TOX7345) and from the third week in the once daily dosing (TOX8394), no further increase in exposure occurred. When normalized to a similar dose level, the exposure (Cₘₐₓ and AUC) was comparable between the two studies.

**Table 2: Pharmacokinetic parameters of bortezomib in plasma calculated after single (day 1) and repeated (day 32, 74 at 0.075 and 0.1 mg/kg or day 50 and 78 at 0.166 mg/kg) subcutaneous administration of bortezomib at 0.075, 0.1 and 0.166 mg/kg in a bi-weekly (0.075 and 0.1 mg/kg) or weekly (0.166 mg/kg) dosing regimen (TOX7345) in Cynomolgus monkeys.**

| Day | Dose (mg/kg) | Tₘₐₓ (h) | Cₘₐₓ (ng/mL) | AUC₀₋₇₂ₕ (h.ng/mL) |
|---|---|---|---|---|
| 1 | 0.075 | 0.117 (0) | 61.1 (10.3) | 36.7 (8.4) |
| 32 | 0.075 | 0.25 (0.20) | 58.8 (29.7) | 110 (5) |
| 74 | 0.075 | 0.139 (0.06) | 70.1 (11.6) | 122 (11) |
| 1 | 0.1 | 0.117 (0) | 86.2 (20.5) | 77.5 (42.2) |
| 32 | 0.1 | 0.119 (0.01) | 141 (19) | 227 (107) |
| 74 | 0.1 | 0.161 (0.07) | 113 (19) | 211 (82) |
| 1 | 0.166 | 0.117 (0.07) | 144 (48) | 160 (17) |
| 50 | 0.166 | 0.117 (0) | 168 (22) | 205 (27) |
| 78 | 0.166 | 0.23 (0.06) | 158 (110) | 229 (157) |

| | | | | |
|---|---|---|---|---|
| Mean values are given. Standard deviations are given between brackets. Tₘₐₓ = Time of occurrence of Cₘₐₓ. Cₘₐₓ = Maximal plasma concentration. AUC₀₋ₜ = Area under the plasma concentration time curve between 0 and t. | | | | |

**Table 3: Pharmacokinetic parameters of bortezomib in plasma calculated after single (day 1) and repeated (day 3 and 54) subcutaneous administration of bortezomib at 0.0166, 0.0333 and 0.05 mg/kg in a daily (5/7 days) dosing regimen for 8 consecutive weeks (TOX8394) in Cynomolgus monkeys.**

| Day | Dose (mg/kg) | Tₘₐₓ (h) | Cₘₐₓ (ng/mL) | AUC₀₋₂₄ₕ (h.ng/mL) |
|---|---|---|---|---|
| 1 | 0.0166 | 0.148 (0.08) | 10.7 (1.9) | 4.78 (1.21) |
| 3 | 0.0166 | 0.138 (0.05) | 11.0 (3.2) | 8.73 (5.04) |
| 54 | 0.0166 | 0.161 (0.07) | 14.1 (2.7) | 20.5 (10.3) |
| 1 | 0.0333 | 0.116 (0.003) | 23.7 (5.2) | 14.4 (6.4) |
| 3 | 0.0333 | 0.165 (0.07) | 27.5 (3.5) | 39.0 (6.0) |
| 54 | 0.0333 | 0.184 (0.07) | 45.5 (6.3) | 85.0 (7.7) |
| 1 | 0.05 | 0.200 (0.155) | 34.2 (8.0) | 30.7 (8.9) |
| 3 | 0.05 | 0.203 (0.07) | 48.1 (10.3) | 64.5 (21.2) |
| 54 | 0.05 | 0.185 (0.07) | 68.3 (12.2) | 101 (15) |

| | | | | |
|---|---|---|---|---|
| Mean values are given. Standard deviations are given between brackets. Tₘₐₓ = Time of occurrence of Cₘₐₓ. Cₘₐₓ = Maximal plasma concentration. AUC₀₋ₜ = Area under the plasma concentration time curve between 0 and t. | | | | |

The results showed that the compound was rapidly absorbed after dosing as the average Tₘₐₓ was between about 0.138 and 0.203 h after dosing. The exposure, represented by Cₘₐₓ as well AUC, increased with increasing dose levels of bortezomib. The maximal proteasome inhibition was observed at about 0.5 h after dosing. The proteasome inhibition also increased with increasing doses of bortezomib.

Similar systemic toxicity in GI, bone marrow, lymphatic system, kidney was observed in both the bi-weekly (TOX7345) and the daily (TOX8394) administration. Also, similar neuropathy was observed in both studies. The local tolerance was very good in both toxicity studies. The daily exposure (AUC) was lower in the once daily dosing at about 0.6 mg/m² than those of the bi-weekly dosing at about 1.2 mg/m². However, the cumulative weekly exposure was higher in the once daily dosing regimen than the biweekly dosing regimen.

The maximal proteasome inhibition (Emax) was similar at about 0.6 mg/m² and 1.2 mg/m² varying between about 65 and 73%. The AUE was lower for about 0.6 mg/m² (AUE₀₋₂₄ₕ: 900 %.h) in the once daily dosing than for about 1.2 mg/m² (AUE₀₋₇₂ₕ:1500 %.h) in the bi-weekly dosing. The cumulative weekly AUE was superior for the daily schedule: 4500 versus 3000 %.h. In the daily dosing schedule, a 2 day rest period appeared to be adequate for pharmacodynamic recovery, without accumulation of proteasome inhibition.

Despite this higher pharmacodynamic exposure in the daily dosing schedule, systemic toxicity was similar for the once daily dosing schedule at about 0.6 mg/m² than for those of the bi-weekly dosing at about 1.2 mg/m².

### EXAMPLE 4. PRECLINICAL PHARMACODYNAMICS OF PROTEASOME INHIBITION

For each study, the 20S proteasome activity in whole blood was determined, as described in Lightcap, Clin. Chem., 46:673 (2000). The chymotryptic activity was assayed by measuring the rate at which the proteasome hydrolyzed an amide bond in a small fluorescence tagged polypeptide substrate. The specific activity (Spa) was normalized to the amount of protein present in the lysate.

For each treatment cycle, the Spa of each time point was calculated as a percentage of the respective baseline value of each individual animal. The time to maximum inhibitory effect (Tmax), the maximum inhibitory effect (Emax) was determined by visual inspection of the data. The Area under the Effect (AUE) curve was estimated by the linear trapezoidal rule.

In both safety evaluation studies, plasma samples were analyzed using a validated LC/MS/MS method with a lower quantification limit of about 0.5 ng/ml.

The pharmacodynamic parameters of bortezomib in the bi-weekly dosing and the once daily dosing regimens were summarized in Tables 4 and 5, respectively. Further, the percentage of proteasome inhibition in whole blood by bortezomib in the bi-weekly dosing and the once daily dosing regimens were shown in Figures 3 and 4, respectively.

The results indicate that there was no gender difference in the bortezomib-mediated proteasome inhibition of 20S proteasome activity. The results also indicate that the maximal inhibition occurred on average at about 0.5 h after subcutaneous administration in both studies.

In each experiment, the inhibition of the 20S proteasome specific inhibition was related with the dose. For the bi-weekly dosing regimen the strongest inhibition was observed for the 0.1 mg/kg dose level (Emax=74 %). For the daily dosing schedule, the highest inhibition was observed at the 0.05-mg/kg dose level (Emax=73 %). For the weekly (0.166 mg/kg) dose group a maximal inhibition of 78 % was noted.

Overall, the course of the inhibition profiles as a function of time after dosing was comparable between the bortezomib dosed groups in each experiment and also between the experiments. However there was a tendency for a slower return of the proteasome inhibition back to the base line for the highest dose levels in particular the 0.166 mg/kg dose level. For the bi-weekly dosage regimen (TOX7345), the proteasome inhibition returned to baseline values between 24 and 48 h after dosing for all groups.

In the daily dosing study (TOX8394), the 20S proteasome activity determined on various days before dosing showed that there was no indication for remaining 20S proteasome inhibition at the end of each 2-day drug-holiday.

The maximal proteasome inhibition (Eₘₐₓ) in blood was similar at 0.05 mg/kg (0.6 mg/m²) daily dosing and 0.1 mg/kg (1.2 mg/ m²) bi-weekly dosing (between 65 and 73 % inhibition). Although the Area Under the Effect Curve (AUE) was lower for the 0.05 mg/kg than for the 0.1 mg/kg between each respective dosing interval, the cumulative AUE (calculated over 1 week) was about 50 % higher for the daily treatment schedule than for the bi-weekly administration.

**Table 4: Pharmacodynamic parameters of bortezomib in whole blood calculated after single (day 1) and repeated (day 32, 74 at 0.075 and 0.1 mg/kg or day 50 and 78 at 0.166 mg/kg) subcutaneous administration of bortezomib at 0.075, 0.1 and 0.166 mg/kg in a bi-weekly (0.075 and 0.1 mg/kg) or weekly (0.166 mg/kg) dosing regimen (TOX7345) in Cynomolgus monkeys.**

| Day | Dose (mg/kg) | Tₘₐₓ (h) | Eₘₐₓ (%) | AUE₀₋₂₄ₕ (% Inhibition.time) |
|---|---|---|---|---|
| 1 | 0.075 | 0.5 | 63 | 585 (87) |
| 32 | 0.075 | 0.5 | 68 | 1086 (158) |
| 74 | 0.075 | 0.5 | 67 | 1111 (116) |
| 1 | 0.1 | 0.5 | 74 | 926 (99) |
| 32 | 0.1 | 0.5 | 71 | 1454 (201) |
| 74 | 0.1 | 0.5 | 72 | 1584 (210) |
| 1 | 0.166 | 1 | 78 | 931 (124) |
| 50 | 0.166 | 0.5 | 78 | 2152 (222) |
| 78 | 0.166 | 1 | 77 | 1468 (219) |

| | | | | |
|---|---|---|---|---|
| Mean values are given. Standard deviations are given between brackets. Tₘₐₓ = Time of occurrence of Eₘₐₓ. Eₘₐₓ = Maximal effect (= % proteasome inhibition). AUE₀₋ₜ = Area under the effect curve between 0 and t. | | | | |

**Table 5: Pharmacodynamic parameters of bortezomib in whole blood on day 3 and day 54 of a subcutaneous administration of bortezomib at 0.0166, 0.0333 and 0.05 mg/kg in a daily (5/7 days) dosing regimen for 8 consecutive weeks (TOX8394) in Cynomolgus monkeys.**

| Day | Dose (mg/kg) | Tₘₐₓ (h) | Eₘₐₓ (%) | AUE₀₋₂₄ₕ (% Inhibition.time) |
|---|---|---|---|---|
| 3 | 0.0166 | 0.5 | 48 | 603 (77) |
| 54 | 0.0166 | 0.5 | 38 | 481 (103) |
| 3 | 0.0333 | 0.5 | 58 | 685 (32) |
| 54 | 0.0333 | 0.5 | 62 | 895 (125) |
| 3 | 0.05 | 0.5 | 65 | 802 (67) |
| 54 | 0.05 | 0.5 | 73 | 1039 (114) |

| | | | | |
|---|---|---|---|---|
| Mean values are given. Standard deviations are given between brackets. Tₘₐₓ = Time of occurrence of Eₘₐₓ. Eₘₐₓ = Maximal effect (= % proteasome inhibition). AUE₀₋ₜ = Area under the effect curve between 0 and t. | | | | |

Despite the higher cumulative dose and cumulative proteasome inhibition, the safety profile was comparable between and bi-weekly dosing and the once daily dosing regimen. The studies also showed that peripheral neuropathy and local tolerance were similar in both dosing regimens in tested animals.

### EXAMPLE 5. CLINICAL STUDIES

Bortezomib is administered subcutaneously once daily on a 5-days on and 2-days off schedule to patients with advanced or metastatic solid tumors, either alone or in combination with radiotherapy. Daily dosing begins on Day 1 of Course 1, and each course is 28 consecutive days. The administered dose is about 0.1, 0.2, 0.3, 0.4, 0.5, 0.6, 0.7, 0.8, 0.9, 1.0, or 1.1 mg/m².

Tumor response is determined for all subjects with measurable lesions using the modified WHO criteria for tumor response. The assessments are made every 2 months or more frequently if indicated.

Thus, while there have been shown and described and pointed out fundamental novel features of the invention as applied to a preferred embodiment thereof, it will be understood that various omissions and substitutions and changes in the form and details of the devices illustrated, and in their operation, may be made by those skilled in the art. It is the intention, therefore, to be limited only as indicated by the scope of the claims appended hereto.

## Claims

1. Bortezomib for use in a method for treating cancer in a patient, comprising administering to said patient multiple doses of said bortezomib, wherein said multiple doses are administered at least four days a week for at least two weeks.

2. Bortezomib for use in a method of claim 1, wherein said bortezomib is administered at least five days a week, or wherein said bortezomib is administered at least six days a week, or wherein said bortezomib is administered at least once daily.

3. Bortezomib for use in a method of claim 1, wherein said multiple doses are administered at least once every 48 hours.

4. Bortezomib for use in a method of claim 3, wherein said multiple doses are administered at least once every 24 hours.

5. Bortezomib for use in a method of any one of claims 1-4, wherein said multiple doses are administered for at least four weeks.

6. Bortezomib for use in a method of any one of claims 1-5, wherein said bortezomib is administered intravenously, subcutaneously, parenterally, intramuscularly, intradermally, or transdermally.

7. Bortezomib for use in a method of any one of claims 1-6, wherein said cancer is a solid tumor.

8. Bortezomib for use in a method of claim 7, wherein said solid tumor is selected from the group consisting of tumors of the skin, lung, bronchus, prostate, breast, pancreas, adrenal gland, small intestine, large intestine, colon, rectum, thyroid, stomach, liver, bile duct, kidney, renal pelvis, urinary bladder, uterus, cervix, ovary, esophagus, brain, head and neck, oral cavity, pharynx, and larynx.

9. Bortezomib for use in a method of any one of claims 1-8, further comprising administering to said patient a second anti-cancer treatment.

10. Bortezomib for use in a method of claim 9, wherein said second anti-cancer treatment is selected from the group consisting of antibody therapy, hormone therapy, radiation, chemotherapy surgery, gene therapy, immunotherapy or other treatments for preventing, treating or ameliorating cancers.

11. Bortezomib for use in a method of claim 10, wherein said chemotherapy is selected from the group consisting of an anti-angiogenic agent, an anti-tumor agent, a cytotoxic agent, and an inhibitor of cell proliferation.

12. Bortezomib for use in a method of any one of claims 1-11, wherein said cancer is refractory or recurrent.

## Patentansprüche

1. Bortezomib zur Verwendung in einem Verfahren zur Behandlung von Krebs bei einem Patienten, umfassend das Verabreichen von multiplen Dosen des Bortezomib an den Patienten, wobei die multiplen Dosen mindestens 4 Tage pro Woche über mindestens 2 Wochen hinweg verabreicht werden.

2. Bortezomib zur Verwendung in einem Verfahren nach Anspruch 1, wobei das Bortezomib mindestens fünf Tage pro Woche verabreicht wird oder wobei das Bortezomib mindestens sechs Tage pro Woche verabreicht wird oder wobei das Bortezomib mindestens einmal pro Tag verabreicht wird.

3. Bortezomib zur Verwendung in einem Verfahren nach Anspruch 1, wobei die multiplen Dosen mindestens einmal alle 48 Stunden verabreicht werden.

4. Bortezomib zur Verwendung in einem Verfahren nach Anspruch 3, wobei die multiplen Dosen mindestens einmal alle 24 Stunden verabreicht werden.

5. Bortezomib zur Verwendung in einem Verfahren nach einem der Ansprüche 1-4, wobei die multiplen Dosen über mindestens vier Wochen hinweg verabreicht werden.

6. Bortezomib zur Verwendung in einem Verfahren nach einem der Ansprüche 1-5, wobei das Bortezomib intravenös, subkutan, parenteral, intramuskulär, intradermal oder transdermal verabreicht wird.

7. Bortezomib zur Verwendung in einem Verfahren nach einem der Ansprüche 1-6, wobei es sich bei dem Krebs um einen festen Tumor handelt.

8. Bortezomib zur Verwendung in einem Verfahren nach Anspruch 7, wobei der feste Tumor aus der Gruppe bestehend aus Tumoren der Haut, der Lunge, des Bronchus, der Prostata, der Brust, des Pankreas, der Nebenniere, des Dünndarms, des Dickdarms, des Kolons, des Rektums, der Schilddrüse, des Magens, der Leber, des Gallengangs, der Niere, des Nierenbeckens, der Harnblase, des Uterus, der Zervix, des Eierstocks, der Speiseröhre, des Hirns, des Kopfes und Halses, der Mundhöhle, des Rachens und des Kehlkopfes ausgewählt ist.

9. Bortezomib zur Verwendung in einem Verfahren nach einem der Ansprüche 1-8, weiterhin umfassend das Verabreichen einer zweiten Antikrebsbehandlung an den Patienten.

10. Bortezomib zur Verwendung in einem Verfahren nach Anspruch 9, wobei die zweite Antikrebsbehandlung aus der Gruppe bestehend aus Antikörpertherapie, Hormontherapie, Bestrahlung, Chemotherapie, chirurgischem Eingriff, Gentherapie, Immuntherapie oder anderen Behandlungen zum Vorbeugen, Behandeln oder Lindern von Krebsarten ausgewählt ist.

11. Bortezomib zur Verwendung in einem Verfahren nach Anspruch 10, wobei die Chemotherapie aus der Gruppe bestehend aus einem antiangiogenen Agens, einem Antitumoragens, einem zytotoxischen Agens und einem Zellproliferationshemmer ausgewählt ist.

12. Bortezomib zur Verwendung in einem Verfahren nach einem der Ansprüche 1-11, wobei der Krebs refraktär oder rekurrent ist.

## Revendications

1. Bortézomib pour utilisation dans un procédé de traitement du cancer chez un patient, comprenant l'administration audit patient de doses multiples dudit bortézomib, où lesdites doses multiples sont administrées au moins quatre jours par semaine pendant au moins deux semaines.

2. Bortézomib pour utilisation dans un procédé de la revendication 1, ledit bortézomib étant administré au moins cinq jours par semaine, ou ledit bortézomib étant administré au moins six jours par semaine, ou ledit bortézomib étant administré au moins une fois par jour.

3. Bortézomib pour utilisation dans un procédé de la revendication 1, lesdites doses multiples étant administrées au moins une fois toutes les 48 heures.

4. Bortézomib pour utilisation dans un procédé de la revendication 3, lesdites doses multiples étant administrées au moins une fois toutes les 24 heures.

5. Bortézomib pour utilisation dans un procédé de l'une quelconque des revendications 1 à 4, lesdites doses multiples étant administrées pendant au moins quatre semaines.

6. Bortézomib pour utilisation dans un procédé de l'une quelconque des revendications 1 à 5, ledit bortézomib étant administré par voie intraveineuse, par voie sous-cutanée, par voie parentérale, par voie intramusculaire, par voie intradermique ou par voie transdermique.

7. Bortézomib pour utilisation dans un procédé de l'une quelconque des revendications 1 à 6, ledit cancer étant une tumeur solide.

8. Bortézomib pour utilisation dans un procédé de la revendication 7, ladite tumeur solide étant choisie dans le groupe constitué de tumeurs de la peau, du poumon, des bronches, de la prostate, du sein, du pancréas, des glandes surrénales, de l'intestin grêle, du gros intestin, du côlon, du rectum, de la thyroïde, de l'estomac, du foie, des canaux biliaires, du rein, du bassinet du rein, de la vessie urinaire, de l'utérus, du col de l'utérus, de l'ovaire, de l'oesophage, du cerveau, de la tête et du cou, de la cavité buccale, du pharynx et du larynx.

9. Bortézomib pour utilisation dans un procédé de l'une quelconque des revendications 1 à 8, comprenant en outre l'administration audit patient d'un deuxième traitement anticancéreux.

10. Bortézomib pour utilisation dans un procédé de la revendication 9, ledit deuxième traitement anticancéreux étant choisi dans le groupe constitué d'une thérapie par anticorps, d'une hormonothérapie, d'un rayonnement, d'une chimiothérapie, d'une chirurgie, d'une thérapie génique, d'une immunothérapie ou d'autres traitements pour la prévention, le traitement ou l'amélioration de cancers.

11. Bortézomib pour utilisation dans un procédé de la revendication 10, ladite chimiothérapie étant choisie dans le groupe constitué d'un agent anti-angiogénique, un agent antitumoral, un agent cytotoxique et un inhibiteur de prolifération cellulaire.

12. Bortézomib pour utilisation dans un procédé de l'une quelconque des revendications 1 à 11, ledit cancer étant réfractaire ou récidivant.
